# EUROPEAN PATENT APPLICATION

(11) **EP 4 567 060 A1**
(43) Date of publication of application: **11.06.2025**
(21) Application number: 24216052.1
(22) Date of filing: 28.11.2024
(51) Int. Cl.: C08K 3/04, C08K 3/36, C08K 3/22, C08K 5/20, B60C 1/00, C07C 15/02, C07C 211/54, C07C 211/55, C07C 233/01

(54) **ANTIOZONANT AND ASSOCIATED RUBBER COMPOSITION AND RUBBER ARTICLE**

(30) Priority: 07.12.2023 US 202318532401
(71) Applicant: The Goodyear Tire & Rubber Company, Akron, OH 44316 (US)
(72) Inventor: MURRAY, Aaron Patrick, Chardon, Ohio, 44024 (US)
(74) Representative: Kutsch, Bernd

(57) **Abstract**

An antiozonant of formula (I) is disclosed.

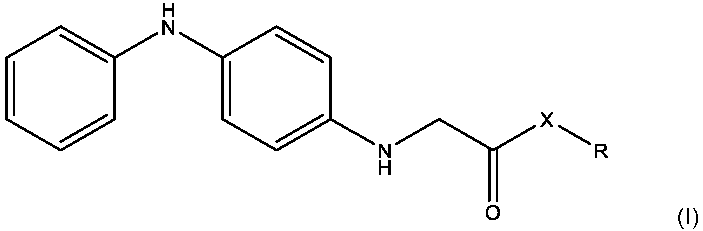

X is selected from the group consisting of O, S, and N. R is selected from the group consisting of hydrogen, an alkyl moiety, a cycloalkyl moiety, an aryl moiety, an amine moiety, an amide moiety, an alcohol moiety, an aldehyde moiety, a ketone moiety, a carboxylic acid moiety, an ether moiety, an ester moiety, and a thiol moiety. A rubber composition contains the antiozonant, a diene elastomer; a reinforcing filler; a sulfur-based curing agent; and an accelerator for the curing agent.

## Description

### BACKGROUND

The present disclosure relates to antiozonants, rubber compositions containing the antiozonants, articles comprising the rubber compositions, and methods of making and using the articles. Non-limiting examples of such articles include tires.

Ozone can attack the surface of rubber, thereby resulting in surface cracks which may develop into deeper and/or larger cracks. Small cracks may negatively impact the physical appearance of rubber articles and larger cracks may shorten the useful lifetime of such articles.

To reduce or eliminate the negative consequences of ozone attack, antiozonants have been developed and added to rubber compositions. One commonly used antiozonant is N-(1,3-dimethylbutyl)-N'-phenyl-p-phenylenediamine or 6PPD which is illustrated below.

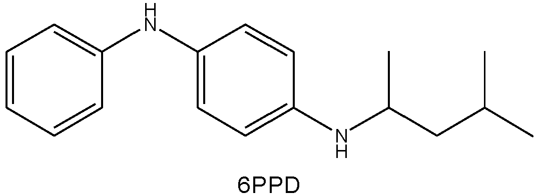

6PPD is known to interact with ozone to form N-(1,3-dimethylbutyl)-N'-phenyl-p-phenylenediamine-quinone or 6PPDQ which is illustrated below.

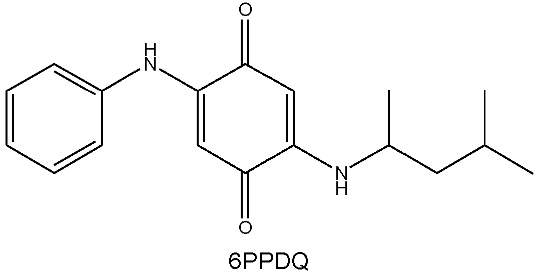

In part due to roadway and stormwater runoff, 6PPDQ may contaminate waterways and research by Tian et al. (Zhenyu Tian et al., A ubiquitous tire rubber-derived chemical induces acute mortality in coho salmon. Science 371,185-189(2021) DOI:10.1126/science.abd6951.) suggests this quinone is highly toxic to Coho salmon.

It would be desirable to develop new antiozonants that do not form 6PPDQ when exposed to ozone.

### SUMMARY OF THE INVENTION

The invention relates to a rubber composition in accordance with claim 1, to a rubber article in accordance with claim 11, to an antiozonant in accordance with claim 13 and to a use in accordance with claim 14.

Dependent claims refer to preferred embodiments of the invention.

In one aspect, the present disclosure relates to antiozonants that do not form 6PPDQ when exposed to ozone, rubber compositions containing the antiozonants, and rubber articles including at least one component formed from the rubber compositions.

Disclosed, in some embodiments, is an antiozonant of formula (I): 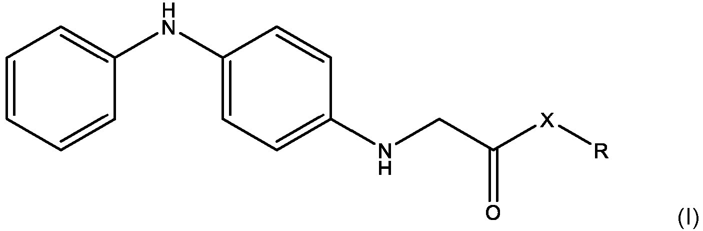 wherein X is selected from the group consisting of O, S, and N; and wherein R is selected from the group consisting of hydrogen, an alkyl moiety, a cycloalkyl moiety, an aryl moiety, an amine moiety, an amide moiety, an alcohol moiety, an aldehyde moiety, a ketone moiety, a carboxylic acid moiety, an ether moiety, an ester moiety, and a thiol moiety.

Disclosed, in other preferred embodiments, is a rubber containing the antiozonant of formula (I); a diene elastomer; a reinforcing filler; a sulfur-based curing agent; and an accelerator for the curing agent.

The antiozonant may be included in the rubber composition in an amount of 0.2 phr to 8 phr per 100 phr of the diene elastomer, including 0.5 phr to 6 phr per 100 phr of the diene elastomer, and 2 phr to 4 phr per 100 phr of the diene elastomer.

In some embodiments, the diene elastomer includes at least one elastomer material selected from the group consisting of polybutadiene rubber (BR), polyisoprene robber (IR), natural rubber (NR), styrene-butadiene robber (SBR), isobutylene isoprene rubber (HR) butadiene copolymers, and isoprene copolymers.

The diene elastomer may include at least one copolymer selected from the group consisting of butadiene-styrene copolymers (SBR), butadiene-isoprene copolymers (BIR), isoprene-styrene copolymers (SIR), and isoprene-butadiene-styrene copolymers (SBIR).

In some embodiments, the reinforcing filler is present in the rubber composition in an amount of 30 phr to 150 phr per 100 phr of the diene elastomer.

The reinforcing filler may include at least one filler material selected from the group consisting of carbon black, silica, graphene, and graphite.

In some embodiments, the reinforcing filler includes carbon black.

The reinforcing filler may further include silica, optionally in combination with a silane coupling agent.

In some embodiments, the rubber composition further includes zinc oxide and/or a fatty acid.

Disclosed, in further embodiments, is a rubber article formed from the rubber composition.

The rubber article may be a tire or a tire component.

These and other non-limiting aspects and/or objects of the disclosure are more particularly described below.

### BRIEF DESCRIPTION OF THE DRAWINGS

The following is a brief description of the drawings, which are presented for the purposes of illustrating the exemplary embodiments disclosed herein.
FIG. 1 is a partial side view of a non-limiting example of a tire in accordance with some embodiments of the present disclosure.
FIG. 2 is a flow chart illustrating an example of an article formation process in accordance with some embodiments of the present disclosure.

### DETAILED DESCRIPTION OF PREFERRED EMBODIMENTS

The present disclosure may be understood more readily by reference to the following detailed description of desired embodiments included therein and the drawings. In the following specification and the claims which follow, reference will be made to a number of terms which shall be defined to have the following meanings.

Unless otherwise defined, all technical and scientific terms used herein have the same meaning as commonly understood by one of ordinary skill in the art. In case of conflict, the present document, including definitions, will control. Methods and materials are described below, although methods and materials similar or equivalent can be used in practice or testing of the present disclosure.

As used in the specification and in the claims, the term "comprising" may include the embodiments "consisting of" and "consisting essentially of." The terms "comprise(s)," "include(s)," "having," "has," "can," "contain(s)," and variants thereof, as used herein, are intended to be open-ended transitional phrases that require the presence of the named ingredients/steps and permit the presence of other ingredients/steps. However, such description should be construed as also describing compositions, mixtures, or processes as "consisting of" and "consisting essentially of" the enumerated ingredients/steps, which allows the presence of only the named ingredients/steps, along with any impurities that might result therefrom, and excludes other ingredients/steps.

In the description, the term "phr" refers to parts of a respective material per 100 parts by weight of rubber, or elastomer. The term "phf" refers to parts per hundred filler, by weight. By convention, the uncured rubber employed in the rubber composition totals 100 phr.

The terms "rubber" and "elastomer" may be used interchangeably unless otherwise indicated. The terms "cure" and "vulcanize" may be used interchangeably unless otherwise indicated.

The present disclosure relates to antiozonants that do not form 6PPDQ upon exposure to ozone. The antiozonants are of the general formula (I) 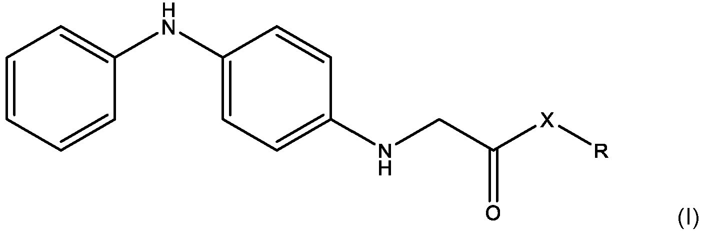 wherein X is selected from the group consisting of O, S, and N; and wherein R is selected from the group consisting of hydrogen, an alkyl moiety, a cycloalkyl moiety, an aryl moiety, an amine moiety, an amide moiety, an alcohol moiety, an aldehyde moiety, a ketone moiety, a carboxylic acid moiety, an ether moiety, an ester moiety, and a thiol moiety. In some embodiments, X is O or S. In particular embodiments, X is O. In some embodiments, R is selected from an aryl moiety, an alkyl moiety, and a cycloalkyl moiety. In particular embodiments, R is an aryl moiety. Both substituted and unsubstituted moieties are encompassed. Moreover, R may be an alkyl, cycloalkyl, or aryl that bears one or more additional functional groups that are tolerant for the cyclization reaction. Non-limiting examples of additional functional groups include ketones, esters, nitriles, and halogens (e.g., F, Cl, Br, I). For example, R may be a substituted alkyl moiety. In a particular embodiment, -X-R is -O-CH₂-CH₂-ketone. Table 1 (below) summarizes various possible combinations of X and R.

**Table 1**

| Compound Number | X | R |
|---|---|---|
| 1 | O | hydrogen |
| 2 | O | an alkyl moiety |
| 3 | O | a cycloalkyl moiety |
| 4 | O | an aryl moiety |
| 5 | O | an amine moiety |
| 6 | O | an amide moiety |
| 7 | O | an alcohol moiety |
| 8 | O | an aldehyde moiety |
| 9 | O | a ketone moiety |
| 10 | O | a carboxylic acid moiety |
| 11 | O | an ether moiety |
| 12 | O | an ester moiety |
| 13 | O | a thiol moiety |
| 14 | S | hydrogen |
| 15 | S | an alkyl moiety |
| 16 | S | a cycloalkyl moiety |
| 17 | S | an aryl moiety |
| 18 | S | an amine moiety |
| 19 | S | an amide moiety |
| 20 | S | an alcohol moiety |
| 21 | S | an aldehyde moiety |
| 22 | S | a ketone moiety |
| 23 | S | a carboxylic acid moiety |
| 24 | S | an ether moiety |
| 25 | S | an ester moiety |
| 26 | S | a thiol moiety |
| 27 | N | hydrogen |
| 28 | N | an alkyl moiety |
| 29 | N | a cycloalkyl moiety |
| 30 | N | an aryl moiety |
| 31 | N | an amine moiety |
| 32 | N | an amide moiety |
| 33 | N | an alcohol moiety |
| 34 | N | an aldehyde moiety |
| 35 | N | a ketone moiety |
| 36 | N | a carboxylic acid moiety |
| 37 | N | an ether moiety |
| 38 | N | an ester moiety |
| 39 | N | a thiol moiety |

In an ozone-containing environment, the antiozonant of formula (I) can form the intermediate of formula (II) and the products of formula (III) instead of forming 6PPDQ.

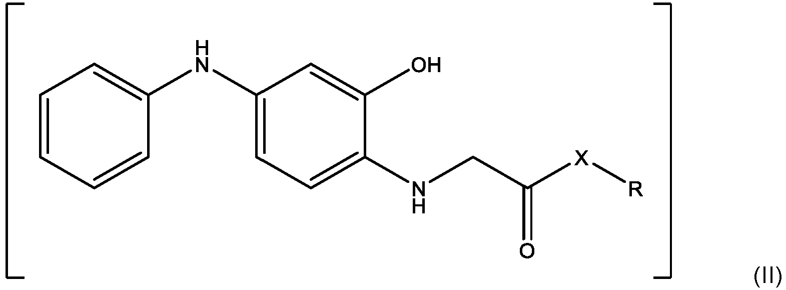

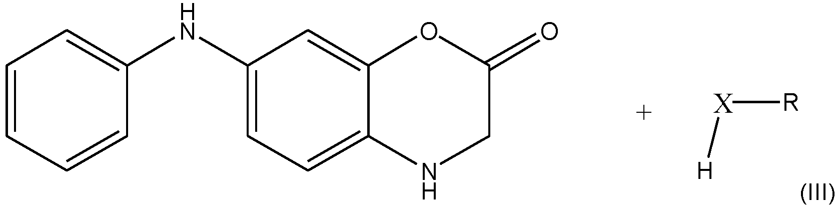

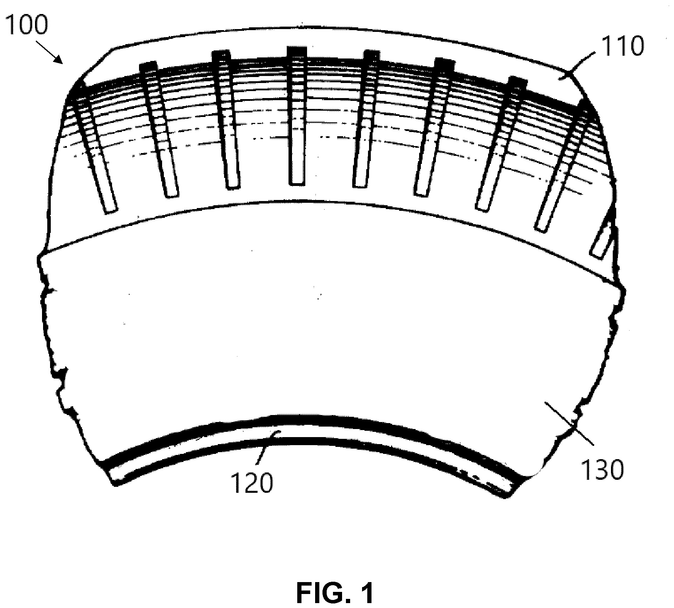

FIG. 1 is a partial side view of a non-limiting example of a tire 100 in accordance with some embodiments of the present disclosure. The tire 100 includes a tread portion 110, a bead portion 120, and a sidewall portion 130 located between the tread portion 110 and the bead portion 120. The tread portion 110, bead portion 120, and sidewall portion 130 of tires in accordance with the present disclosure are not limited to the particular configuration depicted in FIG. 1. For example, there may be multiple sidewall portions, ribs, patterns, colors, etc. The antiozonant of the present disclosure may be utilized in any or all of the tire portions. The pneumatic tire may be a race tire, passenger tire, aircraft tire, agricultural, earthmover, off-the-road, truck tire, and the like. In one embodiment, the tire is a passenger or truck tire. The tire may also be a radial tire or bias tire.

The antiozonant may also be used in other rubber products, including but not limited to non-pneumatic tires, belts, hoses, shoes, air springs, engine mounts, and the like.

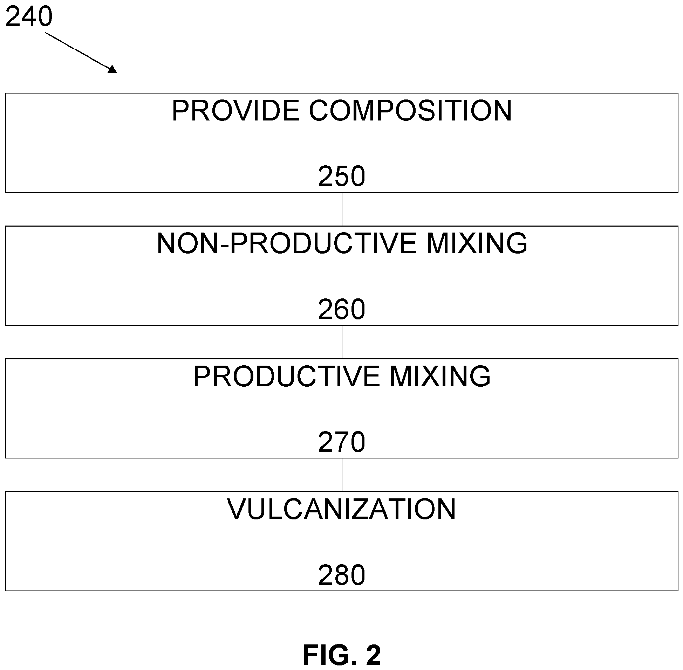

FIG. 2 is a flow chart illustrating an example of a process 240 of forming an article (e.g., a tire) in accordance with some embodiments of the present disclosure. The process 240 includes providing a rubber composition 250, non-productive mixing 260, productive mixing 270, and vulcanization 280. The antiozonant is generally introduced in the non-productive mixing stage 260. However, it is also possible to introduce the antiozonant in the productive mixing stage 270 in addition or as an alternative to introduction in the non-productive mixing stage 260.

### The Rubber Composition

The exemplary rubber composition includes an elastomer component, including at least one conjugated diene; a reinforcing filler component, the antiozonant; and a cure package including a sulfur-based curing agent.

### The Elastomer Component

The exemplary rubber composition includes 100 phr of elastomer(s), including at least one conjugated diene elastomer.

Examples of diene elastomers include polybutadiene rubber (BR), polyisoprene robber (IR), natural rubber (NR), styrene-butadiene robber (SBR), isobutylene isoprene rubber (HR) butadiene copolymers, isoprene copolymers and mixtures of these elastomers. Polyisoprenes include synthetic cis- 1,4 polyisoprene, which may be characterized as possessing cis- 1,4 bonds at more than 90 mol.% or alternatively, at more than 98 mol.%.

Examples of elastomeric copolymer materials include butadiene-styrene copolymers (SBR), butadiene-isoprene copolymers (BIR), isoprene-styrene copolymers (SIR) and isoprene- butadiene-styrene copolymers (SBIR) and mixtures thereof.

In some embodiments, at least 60 phr, or at least 80 phr, or at least 90 phr, or at least 95 phr, or up to 100 phr of the elastomer component is conjugated diene elastomer(s). In some embodiments, the conjugated diene elastomer is selected from natural rubber, a synthetic cis-1,4-polyisoprene, and mixtures thereof. In particular embodiments, the elastomer(s) consist of or consist essentially of natural rubber.

The glass transition temperature (T_{g}) of an elastomer or elastomer composition, where referred to herein, represents the glass transition temperature(s) of the respective elastomer or elastomer composition in its uncured state or possibly a cured state in the case of an elastomer composition. T_{g} values referred to herein are determined as a peak midpoint by a differential scanning calorimeter (DSC) at a temperature rate of increase of 10° C per minute, according to ASTM D3418-21, "Standard Test Method for Transition Temperatures and Enthalpies of Fusion and Crystallization of Polymers by Differential Scanning Calorimetry."

Natural rubber is derived predominantly from isoprene (2-methyl-1,3-butadiene) and thus is predominantly (e.g., at least 99 wt.%) polyisoprene, particularly cis-1,4-polyisoprene. As used herein, the term "natural rubber" means naturally-occurring rubber, such as can be harvested from sources such as Hevea rubber trees and non-Hevea sources (e.g., guayule shrubs and dandelions such as TKS). In other words, the term "natural rubber" should be construed so as to exclude synthetic polyisoprene. Natural rubber produced from field grade coagulum (cuplump) from the Hevea brasiliensis tree is a common form of natural rubber. For example, technically specified or block rubber (TSR), is a natural rubber available from Indonesia, where it may be referred to as Standard Indonesian Rubber (SIR), Malaysia (SMR), and Thailand (STR). It can be obtained in a number of grades, including TSR 10 and TSR 20, with the TSR 10 grade being of higher purity.

In some embodiments, the rubber composition includes at least 60 phr of natural rubber, or at least 80 phr, or at least 90 phr, or at least 92 phr, or at least 95 phr, or at least 98 phr, or up to 100 phr of natural rubber.

Natural rubber is desirable for tear properties (high tear strength) of the cord reinforced rubber composite, low hysteresis (relatively high hot rebound property), and good processability. However, a small amount of synthetic polyisoprene aids green tack properties. Synthetic polyisoprene, in particular, cis-1,4-polyisoprene with a cis content of at least 94 wt. %, may be employed. The cis-1,4-content of the synthetic polyisoprene may be at least 96 wt. %. The T_{g} of the synthetic polyisoprene may be in a range of - 60°C to -70°C.

In some embodiments, synthetic polyisoprene is absent from the rubber composition. In another embodiment, the rubber composition includes at least 2 phr synthetic polyisoprene, or at least 3 phr, or at least 4 phr, or at least 6 phr, or at least 10 phr, or up to 20 phr synthetic polyisoprene.

Other elastomers which may be present, in a total amount of up to 40 phr, or up to 20 phr, or up to 10 phr may be selected from the homopolymerization products of butadiene and its homologues and derivatives, for example, methyl butadiene, dimethylbutadiene, and pentadiene and copolymers formed from butadiene or its homologues or derivatives with other unsaturated monomers. Among the latter may be acetylenes, for example, vinyl acetylene, olefins, for example, isobutylene, which copolymerizes with isoprene to form butyl rubber, vinyl compounds, for example, acrylic acid, acrylonitrile (which polymerize with butadiene to form NBR), methacrylic acid and styrene, the latter compound polymerizing with butadiene to form SBR, as well as vinyl esters and various unsaturated aldehydes, ketones and ethers, e.g., acrolein, methyl isopropenyl ketone and vinylethyl ether. Specific examples of such synthetic rubbers include neoprene (polychloroprene), polybutadiene (including cis 1,4-polybutadiene), butyl rubber, halobutyl rubber, such as chlorobutyl rubber and bromobutyl rubber, styrene/isoprene/butadiene rubber, copolymers of 1,3-butadiene or isoprene with monomers such as styrene, acrylonitrile and methyl methacrylate, ethylene/propylene terpolymers, also known as ethylene/propylene/diene monomers (EPDM), and in particular, ethylene/propylene/dicyclopentadiene terpolymers. Additional examples of elastomers which may be used include alkoxy-silyl end functionalized solution polymerized polymers (SBR, PBR, IBR and SIBR), and silicon-coupled and tin-coupled star-branched polymers.

### Reinforcing fillers

In some embodiments, the rubber composition includes carbon black.

Representative examples of carbon blacks include N110, N121, N134, N220, N231, N234, N242, N293, N299, N315, N326, N330, N332, N339, N343, N347, N351, N358, N375, N539, N550, N582, N630, N642, N650, N683, N754, N762, N765, N774, N787, N907, N908, N990 and N991. These carbon blacks have iodine absorptions ranging from 9 to 145 g/kg and DBP number ranging from 34 cm³/100 g to 150 cm³/100 g.

The carbon black included in the rubber compositions produced by the methods disclosed herein may, in particular embodiments for example, be in an amount of between 30 phr and 150 phr or alternatively between 40 phr and 100 phr or between 40 phr and 80 phr. Suitable carbon blacks are any carbon blacks known in the art and suitable for the given purpose for example, any carbon black having a BET surface area or a specific CTAB surface area both of which are less than 400 m²/g or alternatively, between 20 and 200 m²/g may be suitable for particular embodiments based on the desired properties of the cured rubber composition. The CTAB specific surface area is the external surface area determined in accordance with Standard AFNOR-NFT-45007 of November 1987. Suitable carbon blacks of the type HAF, ISAF and SAF, for example, are conventionally used in tire treads.

Silica may be useful as reinforcement filler in addition to or as an alternative to carbon black. The silica may be any reinforcing silica known to one having ordinary skill in the art including, for example, any precipitated or pyrogenic silica having a BET surface area and a specific CTAB surface area both of which are less than 450 m²/g or alternatively, between 20 and 400 m²/g may be suitable for particular embodiments based on the desired properties of the cured rubber composition. Particular embodiments of rubber compositions disclosed herein may include a silica having a CTAB of between 80 and 200 m²/g, between 100 and 190 m²/g, between 120 and 190 m²/g or between 140 and 180 m²/g.

Silica may be used in combination with a silane coupling agent. Specific examples of silane coupling agents include alkoxyorganomercaptosilanes and bis(3-triethoxysilylpropyl)polysulfide containing an average of from 1 to 5 connecting sulfur atoms (preferably 2 to 4) in its polysulfidic bridge. Bis(3-triethoxysilylpropyl)polysulfide having an average of from 2 to 2.6 and from 3.4 to 3.8, respectively, connecting sulfur atoms in its polysulfidic bridge may be obtained as Si266 ^{™} and Si69 ^{™} from Evonik Industries.

The rubber composition may include at least 0.1 phr, or at least 0.5 phr or at least 1 phr, or up to 6 phr, or up to 3 phr, or up to 2 phr of the silica coupling agent. The silica coupling agent may be present in an amount sufficient to provide a ratio, by weight, of the total amount of coupling agent to silica filler of at least 0.1:100, or at least 1:100, or at least 2:100, or up to 25:100, or up to 20:100, or up to 8:100. When the silica is pretreated with the coupling agent, lower amounts of coupling agent may be used than when the silica is treated in situ with the coupling agent.

Examples of silica dispersing aids include glycols, such as fatty acids, diethylene glycols, alkylene glycols such as polyethylene glycols and polypropylene glycols, fatty acid esters of hydrogenated or non-hydrogenated C₅ or C₆ sugars, polyoxyethylene derivatives of fatty acid esters of hydrogenated or non-hydrogenated C₅ or C₆ sugars, and mixtures thereof. Exemplary fatty acids include stearic acid, palmitic acid, and oleic acid. Exemplary fatty acid esters of hydrogenated and non-hydrogenated C₅ and C₆ sugars (e.g., sorbose, mannose, and arabinose) include sorbitan oleates, such as sorbitan monooleate, dioleate, trioleate and sesquioleate, and sorbitan esters of laurate, palmitate, and stearate fatty acids. Exemplary polyoxyethylene derivatives of fatty acid esters of hydrogenated and non-hydrogenated C₅ and C₆ sugars include polysorbates and polyoxyethylene sorbitan esters, which are analogous to the fatty acid esters of hydrogenated and non-hydrogenated sugars except that ethylene oxide groups are placed on each of the hydroxyl groups.

Exemplary polyalkylene oxides may have a weight average molecular weight Mw of at least 500, such as at least 2,000, or at least 4,000, or up to 15,000, or up to 12,000, or up to 10,000, or up to 8,500. In cases where polyalkylene oxide, e.g., polyethylene glycol, is utilized in the rubber composition, it may be included in an amount of at least 0.1 phr, such as at least 0.2 phr, or at least 0.3 phr, or up to 5 phr, or up to 3 phr, or up to 2 phr, or up to 1 phr, or up to 0.6 phr.

Examples of commercially available polyethylene glycols include Carbowax^{™} PEG 3350 and Carbowax^{™} PEG 8000 from the Dow Chemical Company where the number indicates an approximate weight average molecular weight. Other polyalkylene oxide polymers may be used, as described, for example, in U.S. Pat. Nos 6,322,811 and 4,082,703.

Silica dispersing aids, if used, may be present in a total amount of at least 0.1 wt. %, based on the weight of the silica, or at least 0.5 wt. %, or at least 1 wt. %, or up to 25 wt. %, or up to 20 wt. % or up to 15 wt. %.

Other fillers may also be included as reinforcing fillers to the elastomer system, for example, graphene, graphite, zeolite, calcium carbonate, alumina, aluminum hydroxide, clay (reinforcing grades), magnesium hydroxide, boron nitride, aluminum nitride, titanium dioxide, reinforcing zinc oxide, and combinations thereof.

### Other Compounding ingredients

The rubber composition may include additional components such as antidegradants, processing aids, peptizing agents, sulfur-based curing agents, activators, cure accelerators, and cure retardants.

Representative antidegradants include monophenols, bisphenols, thiobisphenols, polyphenols, hydroquinone derivatives, phosphites, phosphate blends, thioesters, naphthylamines, diphenol amines as well as other diaryl amine derivatives, para-phenylene diamines, quinolines, and blended amines. Exemplary antioxidants include 2,2,4-trimethyl-1,2-dihydroquinoline (TMQ), , and others, such as those disclosed in The Vanderbilt Rubber Handbook (1978), pages 344 346.

Exemplary processing aids include liquid plasticizers, waxes, resins, and combinations thereof. Liquid plasticizers, such as processing oils, have a T_{g} that below 0°C, generally well below, such as less than -30°C, or less than -40°C, or less than - 50°C, such as a T_{g} of 0°C to -100°C. Processing oil, in relatively low concentrations, aids in mixing the ingredients for the rubber composition and/or promotes the processing of the rubber composition. Representative processing oils which may be used in the rubber composition include aliphatic oils, aromatic oils, naphthenic oils, triglyceride oils, low polycyclic aromatics (PCA) oils, such as mild extract solvate (MES), treated distillate aromatic extract (TDAE), special residual aromatic extract (SRAE), and heavy naphthenic oils, and mixtures thereof. Triglyceride oils that can be used include vegetable oils, such as castor oil, soybean oil, canola oil (rapeseed oil), corn oil, cottonseed oil, olive oil, palm oil, safflower oil, sunflower oil, coconut oil, and peanut oil. Castor oil is a triglyceride oil that contains approximately 87 wt. % ricinoleic acid, 7 wt. % oleic acid, 3 wt. % linoleic acid, 2 wt. % palmitic acid, and 1 wt. % stearic acid. Example naphthenic processing oils include Hyprene^{™} 100 from Ergon Refining, Inc., and Tufflo 100^{™} from the Barton Solvent Company. The processing oil(s) may be used in the rubber composition at from 0 to 30 phr, such as at least 0.5 phr, or at least 1 phr, or up to 10 phr, or up to 7 phr, or up to 5 phr, or up to 3 phr. In some embodiments, an oil may be used as a carrier for one or more other components, such as for the elemental sulfur.

Exemplary resins which may be used in the rubber composition are generally solid or viscous at room temperature and include tackifying resins, such as unreactive phenol formaldehyde, and stiffness resins, such as reactive phenol formaldehyde resins and resorcinol or resorcinol and hexamethylene tetramine, hexa(methoxymethyl) melamine (HMMM) resins, benzoxazine resins, and hydrocarbon resins. Example hydrocarbon resins include aromatic, aliphatic, and cycloaliphatic resins.

In some embodiments the rubber composition includes 1 to 10 phr of resin. In another embodiment, the rubber composition is free or substantially free of resin. This is a benefit because resins can constitute potential threats to environment, health, and safety during mixing and/or tire manufacturing. Thus, identifying compositions with good adhesion and/or stiffness properties while avoiding resins is of interest. In particular, the amount of resin in the composition may be less than 1 phr, or less than 0.5 phr, or less than 0.2 phr or 0 phr.

Suitable waxes, particularly microcrystalline waxes, may be of the type described in The Vanderbilt Rubber Handbook (1978), Pages 346 and 347. Waxes may be absent from the rubber composition or composition or used in an amount of at least 1 phr or up to 5 phr.

Exemplary peptizing agents include pentachlorophenol dibenzamidodiphenyl disulfide. Peptizing agents may be absent from the rubber composition or used in an amount of at least 0.1 phr, or up to 1 phr, or up to 0.4 phr.

Exemplary sulfur-based curing agents include elemental sulfur (free sulfur) and sulfur-donating curing agents, such as amine disulfides, polymeric polysulfides, and sulfur olefin adducts. The amount of sulfur-based curing agent may vary, depending on the type of rubber and particular type of curing agent, but may range from 0.1 phr to 10 phr, such as at least 0.5 phr, or at least 1 phr, or at least 2 phr, or up to 8 phr, or up to 6 phr.

Exemplary activators for sulfur curing agents include fatty acids and zinc oxide. The zinc oxide may help to improve adhesion between metal wire and the coating rubber compound upon sulfur curing. The rubber composition may include from 1 phr to 20 phr of zinc oxide, such as at least 5 phr, or at least 7 phr, or up to 15 phr, or up to 12 phr, or up to 10 phr of zinc oxide. Exemplary fatty acids include stearic acid and mixtures of stearic acid with other fatty acids. The rubber composition may include from 0.5 phr of fatty acid, such as at least 0.7 phr, or at least 1 phr, or at least 1.2 phr, or up to 10 phr, or up to 5 phr, or up to 2 phr of fatty acid.

Accelerators may be used to control the time and/or temperature required for vulcanization and to improve the properties of the vulcanizate. The accelerator may be at least 0.2 phr, or at least 0.5 phr, or at least 1 phr, or up to 5 phr, or up to 3 phr in the rubber composition.

Exemplary accelerators for the sulfur-based curing agent include amines, guanidines, thioureas, thiols, thiazoles, thiurams, sulfenamides, dithiocarbamates and xanthates. Examples of thiazole cure accelerators include 2-mercaptobenzothiazole, 2,2'-dithiobis(benzothiazole) (MBTS), N-cyclohexyl-2-benzothiazole-sulfenamide (CBS), and N-tert-butyl-2-benzothiazole-sulfenamide (TBBS), guanidine vulcanization accelerators, such as diphenyl guanidine (DPG), and mixtures thereof. In some embodiments, a single accelerator system may be used, i.e., primary accelerator. In other embodiments, a primary accelerator is used in combination with a secondary accelerator, with the primary accelerator becoming active at a lower temperature than the secondary accelerator but losing its activity quicker. The secondary accelerator may be used in a smaller amount than the primary accelerator. The primary accelerator may be a sulfenamide and the secondary accelerator may be a guanidine, dithiocarbamate, or thiuram compound, such as diphenyl guanidine.

As will be appreciated, the rubber composition may include other ingredients than those exemplified herein.

### Forming the rubber composition

The various components of the rubber layer can be mixed together using convenient rubber mixing equipment, such as an internal rubber mixer. In general, the elastomers, e.g., butyl rubber and natural rubber, are blended in at least one non-productive mixing stage, in the absence of the curing agent followed by a final, or productive, mixing stage in which the curing agent (and possibly one or more of the additional ingredients) is added. In the productive mixing stage, the mixing typically occurs at a temperature, or ultimate temperature, lower than the mixing temperature(s) used in the preceding non-productive mix stage(s).

In some embodiments, in a first, non-productive stage (or stages), the components, other than the sulfur-based curing agent, are stirred in a mixer set at a suitable temperature, such as at least 100°C, or at least 110°C, or up to 165 °C. The temperature of the mixture rises as it is stirred. When the temperature reaches 150°C - 165°C, the mixture is dropped from the mixer and allowed to cool before returning it to the mixer. In a second, productive stage, the mixture is returned to the mixer with the curing agent for from 2 to 30 minutes, to incorporate the curing agent into the mixture, but without appreciably curing the mixture.

Vulcanization of a pneumatic tire incorporating the rubber composition may be carried out at a temperature of 100° C to 200° C, such as from 110° C to 180° C. Any of the usual vulcanization processes may be used such as heating in a press or mold, heating with superheated steam or hot air. Such tires can be built, shaped, molded and cured by various methods which are known and will be readily apparent to those having skill in such art.

The rubber composition typically has sufficient viscosity and unvulcanized tack to enable its incorporation into an unvulcanized tire without significantly departing from conventional tire building techniques. In some embodiments, strips of the rubber composition are formed.

### Tire Construction

To form a cord-reinforced rubber component, such as a ply, a plurality of cords, arranged generally in parallel, are coated with the rubber composition, prior to curing the rubber composition. The coated cords may be passed through a calender which applies pressure and optionally heat to provide the ply with relatively smooth upper and lower surfaces defined by the rubber composition.

A ply or plies, formed of the rubber composition described above, may be built into an unvulcanized tubeless pneumatic rubber tire prior to undergoing vulcanization. Also disclosed is a tire including the vulcanized rubber composition.

In some embodiments, the tire includes a ply, e.g., one or more of a belt ply, a carcass ply, an overlay ply (covering one or more belt plies), and a ply strip (which may be spirally wound in a circumferential direction of the tire). The ply comprises the exemplary rubber composition, e.g., in the form of a rubber coating added to a textile or wire material. The ply may be formed in a calender device (e.g., a wire calender or textile calender). The coating comprises or consists of the rubber composition in accordance with one or more of its embodiments described herein.

The tire containing plies formed of the exemplary rubber composition may be a race tire, passenger tire, aircraft tire, agricultural tire, earthmover tire, off-the-road tire, truck tire, or the like. The tire may be a radial or bias-type tire. In some embodiments, the tire is a pneumatic radial (medium) truck tire. WO 2022/146441 A1 discloses certain aspects related to rubber compositions.

## Claims

1. A rubber composition comprising: a diene elastomer; a reinforcing filler; a sulfur-based curing agent; an accelerator for the curing agent; and an antiozonant of formula (I): 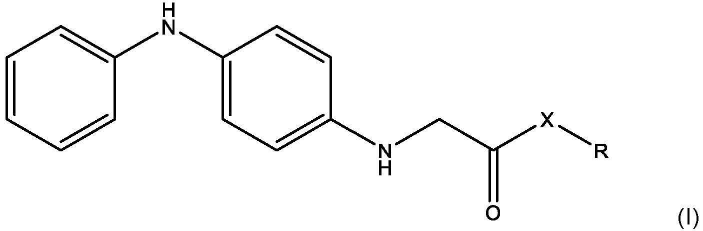 wherein X is selected from the group consisting of O, S, and N; and wherein R is selected from the group consisting of hydrogen, an alkyl moiety, a cycloalkyl moiety, an aryl moiety, an amine moiety, an amide moiety, an alcohol moiety, an aldehyde moiety, a ketone moiety, a carboxylic acid moiety, an ether moiety, an ester moiety, and a thiol moiety.

2. The rubber composition of claim 1, wherein the antiozonant is present in the rubber composition in an amount of 0.2 phr to 8 phr, preferably 0.5 phr to 6 phr or 2 to 4 phr, per 100 phr of the diene elastomer.

3. The rubber composition of claim 1, wherein the antiozonant is present in the rubber composition in an amount of 0.2 phr to 15 phr or from 0.5 phr to 10 phr per 100 phr of the diene elastomer.

4. The rubber composition of claim 1, 2 or 3, wherein the diene elastomer comprises at least one elastomer material selected from the group consisting of polybutadiene rubber (BR), polyisoprene robber (IR), natural rubber (NR), styrene-butadiene robber (SBR), isobutylene isoprene rubber (HR) butadiene copolymers, and isoprene copolymers.

5. The rubber composition of at least one of the previous claims, wherein the diene elastomer comprises at least one copolymer selected from the group consisting of butadiene-styrene copolymers (SBR), butadiene-isoprene copolymers (BIR), isoprene-styrene copolymers (SIR), and isoprene-butadiene-styrene copolymers (SBIR).

6. The rubber composition of at least one of the previous claims, wherein the reinforcing filler is present in the rubber composition in an amount of 30 phr to 150 phr per 100 phr of the diene elastomer.

7. The rubber composition of at least one of the previous claims, wherein the reinforcing filler comprises at least one filler material selected from the group consisting of carbon black, silica, graphene, and graphite.

8. The rubber composition of at least one of the previous claims, wherein the reinforcing filler comprises carbon black and silica.

9. The rubber composition of at least one of the previous claims, further comprising a silane coupling agent.

10. The rubber composition of at least one of the previous claims, further comprising a fatty acid and/or zinc oxide.

11. A rubber article formed from or comprising a rubber composition in accordance with at least one of the previous claims.

12. The rubber article of claim 11, wherein the rubber article is a tire or a tire component.

13. An antiozonant of formula (I): 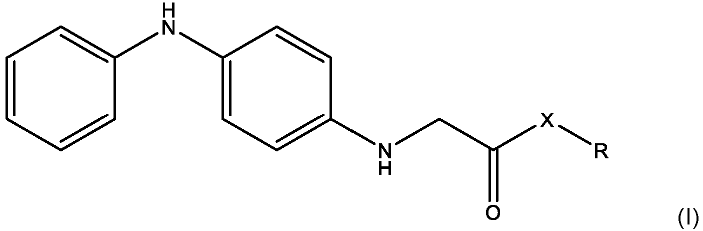 wherein X is selected from the group consisting of O, S, and N; and wherein R is selected from the group consisting of hydrogen, an alkyl moiety, a cycloalkyl moiety, an aryl moiety, an amine moiety, an amide moiety, an alcohol moiety, an aldehyde moiety, a ketone moiety, a carboxylic acid moiety, an ether moiety, an ester moiety, and a thiol moiety.

14. Use of the antiozonant of claim 13 in a rubber article.

15. The use of claim 14 wherein the rubber article is a tire.
